# EUROPEAN PATENT SPECIFICATION

(11) **EP 2 286 811 B1**
(45) Date of publication and mention of the grant of the patent: **05.09.2012**
(21) Application number: 10012138.3
(22) Date of filing: 13.11.2002
(51) Int. Cl.: C07D 211/46, C07D 491/04, A61K 31/4353

(54) **Chemokine receptor antagonists and methods of use thereof**
Chemokine Rezeptor Antagonisten und Methoden zu deren Anwendung
Antagonistes de récepteur de chemokine et leurs procédés d'utilisation

(30) Priority: 21.11.2001 US 989086
(43) Date of publication of application: 23.02.2011
(62) Divisional of application: 06004401.3
(73) Proprietor: Millennium Pharmaceuticals, Inc., Cambridge, MA 02139 (US); Kyowa Hakko Kirin Co., Ltd., Tokyo 100-8185 (JP)
(72) Inventor: Luly, Jay, R., Wellesley, MA 02481 (US); Nakasato, Yoshisuke, Susono-shi Shizuoka (JP); Oshima, Etsuo, Nagareyama-shi Chiba (JP); Harriman, Geraldine, C.B., Charlestown, RI 02813 (US); Carson, Kenneth, G., Princenton New Jersey 02445 (US); Ghosh, Shomir, Brookline, MA 02446 (US); Elder, Amy, M., Arlington, MA 02474 (US); Mattia, Karen, M., Marlborough MA 01752 (US)
(74) Representative: Raynor, Stuart Andrew

(56) References cited:
- WO-A-01/09138
- WO-A-96/31469
- WO-A-99/37651

## Description

### BACKGROUND OF THE INVENTION

Chemoattractant cytokines or chemokines are a family of proinflammatory mediators that promote recruitment and activation of multiple lineages of leukocytes and lymphocytes. They can be released by many kinds of tissue cells after activation. Continuous release of chemokines at sites of inflammation mediates the ongoing migration of effector cells in chronic inflammation. The chemokines characterized to date are related in primary structure. They share four conserved cysteines, which form disulfide bonds. Based upon this conserved cysteine motif, the family is divided into two main branches, designated as the C-X-C chemokines (α-chemokines), and the C-C chemokines (β-chemokines), in which the first two conserved cysteines are separated by an intervening residue, or adjacent respectively (Baggiolini, M. and Dahinden, C. A., Immunology Today, 15:127-133 (1994)).

The C-X-C chemokines include a number of potent chemoattractants and activators of neutrophils, such as interleukin 8 (IL-8), PF4 and neutrophil-activating peptide-2 (NAP-2). The C-C chemokines include RANTES (Regulated on Activation, Normal T Expressed and Secreted), the macrophage inflammatory proteins 1α and 1β (MIP-1α and MIP-1β), eotaxin and human monocyte chemotactic proteins 1-3 (MCP-1, MCP-2, MCP-3), which have been characterized as chemoattractants and activators of monocytes or lymphocytes but do not appear to be chemoattractants for neutrophils. Chemokines, such as RANTES and MIP-1α, have been implicated in a wide range of human acute and chronic inflammatory diseases including respiratory diseases, such as asthma and allergic disorders.

The chemokine receptors are members of a superfamily of G protein-coupled receptors (GPCR) which share structural features that reflect a common mechanism of action of signal transduction (Gerard, C. and Gerard, N.P., Annu Rev. Immunol., 12:775-808 (1994); Gerard, C. and Gerard, N. P., Curr. Opin. Immunol., 6:140-145 (1994)). Conserved features include seven hydrophobic domains spanning the plasma membrane, which are connected by hydrophilic extracellular and intracellular loops. The majority of the primary sequence homology occurs in the hydrophobic transmembrane regions with the hydrophilic regions being more diverse. The first receptor for the C-C chemokines that was cloned and expressed binds the chemokines MIP-1α and RANTES. Accordingly, this MIP-1α/RANTES receptor was designated C-C chemokine receptor 1 (also referred to as CCR-1; Neote, K, et al., Cell, 72:415-425 (1993); Horuk, R, et al., WO 94/11504, May 26, 1994; Gao, J.-I. et al., J. Exp. Med., 177:1421-1427 (1993)). Three receptors have been characterized which bind and/or signal in response to RANTES: CCR3 mediates binding and signaling of chemokines including eotaxin, RANTES, and MCP-3 (Ponath et al., J. Exp. Med., 183:2437 (1996)), CCR4 binds chemokines including RANTES, MIP-1α, and MCP-1 (Power, et al., J. Biol. Chem., 270:19495 (1995)), and CCR5 binds chemokines including MIP-1α, RANTES, and MIP-1β (Samson, et al., Biochem. 35: 3362-3367 (1996)). RANTES is a chemotactic chemokine for a variety of cell types, including monocytes, eosinophils, and a subset of T-cells. The responses of these different cells may not all be mediated by the same receptor, and it is possible that the receptors CCR1, CCR4 and CCR5 will show some selectivity in receptor distribution and function between leukocyte types, as has already been shown for CCR3 (Ponath *et al*.). In particular, the ability of RANTES to induce the directed migration of monocytes and a memory population of circulating T-cells (Schall, T. et al., Nature, 347:669-71 (1990)) suggests this chemokine and its receptor(s) may play a critical role in chronic inflammatory diseases, since these diseases are characterized by destructive infiltrates of T cells and monocytes.

Many existing drugs have been developed as antagonists of the receptors for biogenic amines, for example, as antagonists of the dopamine and histamine receptors. No successful antagonists have yet been developed to the receptors for the larger proteins such as chemokines and C5a. Small molecule antagonists of the interaction between C-C chemokine receptors and their ligands, including RANTES and MIP-1α, would provide compounds useful for inhibiting harmful inflammatory processes "triggered" by receptor ligand interaction, as well as valuable tools for the investigation of receptor-ligand interactions. Compounds which are chemokine receptor antagonists and useful for treating a disease associated with aberrant leukocyte recruitment and/or activation are described in WO 01/09138.

### SUMMARY OF THE INVENTION

It has now been found that a class of small organic molecules are antagonists of chemokine receptor function and can inhibit leukocyte activation and/or recruitment. An antagonist of chemokine receptor function is a molecule which can inhibit the binding and/or activation of one or more chemokines, including C-C chemokines such as RANTES, MIP-1α, MCP-2, MCP-3 and MCP-4 to one or more chemokine receptors on leukocytes and/or other cell types. As a consequence, processes and cellular responses mediated by chemokine receptors can be inhibited with these small organic molecules. Based on this discovery, the treatment of a disease associated with aberrant leukocyte recruitment and/or activation is disclosed as well as the treatment of a disease mediated by chemokine receptor function. The treatment comprises administering to a subject in need an effective amount of a compound or small organic molecule which is an antagonist of chemokine receptor function. Compounds or small organic molecules which have been identified as antagonists of chemokine receptor function are discussed in detail hereinbelow, and can be used for the manufacture of a medicament for treating or for preventing a disease associated with aberrant leukocyte recruitment and/or activation.

The invention also relates to the disclosed compounds and small organic molecules for use in treating or preventing a disease associated with aberrant leukocyte recruitment and/or activation. The invention also includes pharmaceutical compositions comprising one or more of the compounds or small organic molecules which have been identified herein as antagonists of chemokine function and a suitable pharmaceutical carrier. The invention further relates to novel compounds which can be used to treat an individual with a disease associated with aberrant leukocyte recruitment and/or activation and methods for their preparation.

The present invention provides a pharmaceutical composition comprising the (S)-enantiomer and the (R)-enantiomer of 4-(4-Chloro-phenyl)-1-{3-[7-(1-hydroxy-1-methyl-ethyl)-11H-10-oxa-1-aza-dibenzo[a,d]cyclohepten-5-ylidene]-propyl}-3,3-dimethyl-piperidin-4-ol, or a physiologically acceptable salt thereof, wherein the ratio of (S)-enantiomer to (R)-enantiomer (w/w) in the composition is at least about 2:1.

In one embodiment, the ratio of (S)-enantiomer to (R)-enantiomer (w/w) in the composition is at least about 5:1.

In another embodiment, the ratio of (S)-enantiomer to (R)-enantiomer (w/w) in the composition is at least about 10:1.

In a further embodiment, the ratio of (S)-enantiomer to (R)-enantiomer (w/w) in the composition is at least about 20:1.

In an additional embodiment, the ratio of (S)-enantiomer to (R)-enantiomer (w/w) in the composition is at least about 50:1.

Preferably, the pharmaceutical composition comprises a physiologically acceptable carrier or excipient.

Also provided is a pharmaceutical composition of the present invention for use in treating a disease associated with aberrant leukocyte recruitment, activation or recruitment and activation, wherein said disease is selected from the group consisting of arthritis, atherosclerosis, arteriosclerosis, restenosis, ischemia/reperfusion injury, diabetes mellitus, psoriasis, multiple sclerosis, inflammatory bowel disease, rejection of a transplanted organ or tissue, graft versus host disease, allergy and asthma.

### BRIEF DESCRIPTION OF THE DRAWINGS

Figure 1 is a schematic showing the preparation of the compounds represented by Structural Formula (I).
Figure 2 is a schematic showing the preparation of the compounds represented by Compound (VI-b).
Figure 3 is a schematic showing the preparation of the compounds represented by Structural Formula (I)
Figure 4 is a schematic showing the preparation of the compounds represented by Structural Formula (I), wherein Z is represented by Structural Formula (III) and wherein Ring B in Z is substituted with R⁴⁰.
Figure 7 shows the preparation of compounds represented by Structural Formula (I), where in Z is represented by Structural Formulas (III) and wherein Ring B in Z is substituted with R⁴⁰.

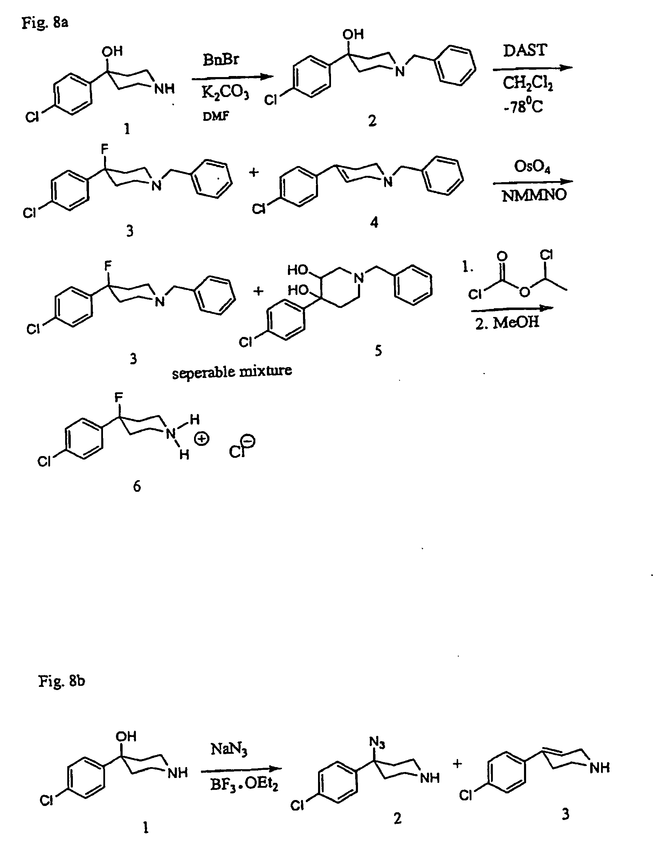

Figure 8A is a schematic showing the preparation of 4-(4-chlorophenyl)-4-fluoropiperidine.
Figure 8B is a schematic showing the preparation of 4-4-azido-4-(4-chlorophenyl)piperidine.

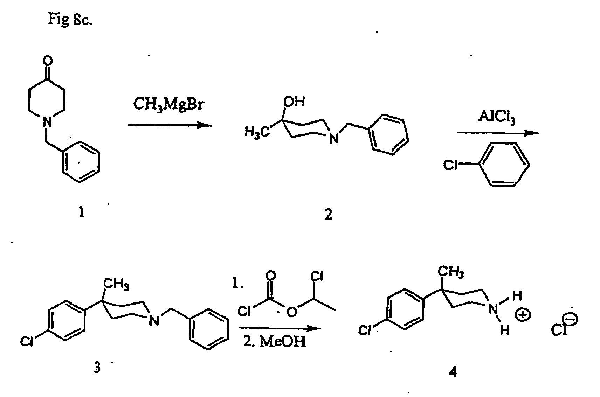

Figure 8C is a schematic showing the preparation of 4-(4-chlorophenyl)-4-methylpiperidine.

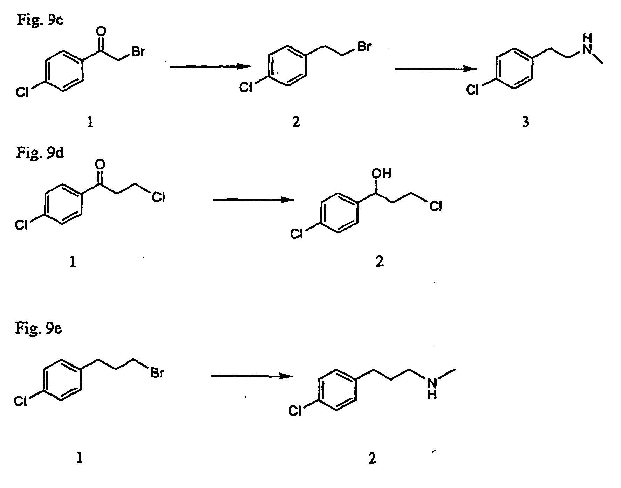

Figure 9C is a schematic showing the preparation of 2-(4-chlorophenyl)-1-(*N-*methyl)ethylamine.
Figure 9D is a schematic showing the preparation of 3-(4-chlorophenyl)-3-chloro-1-hydroxypropane.
Figure 9E is a schematic showing the preparation of 3-(4-chlorophenyl)-1-*N-*methylaminopropane.

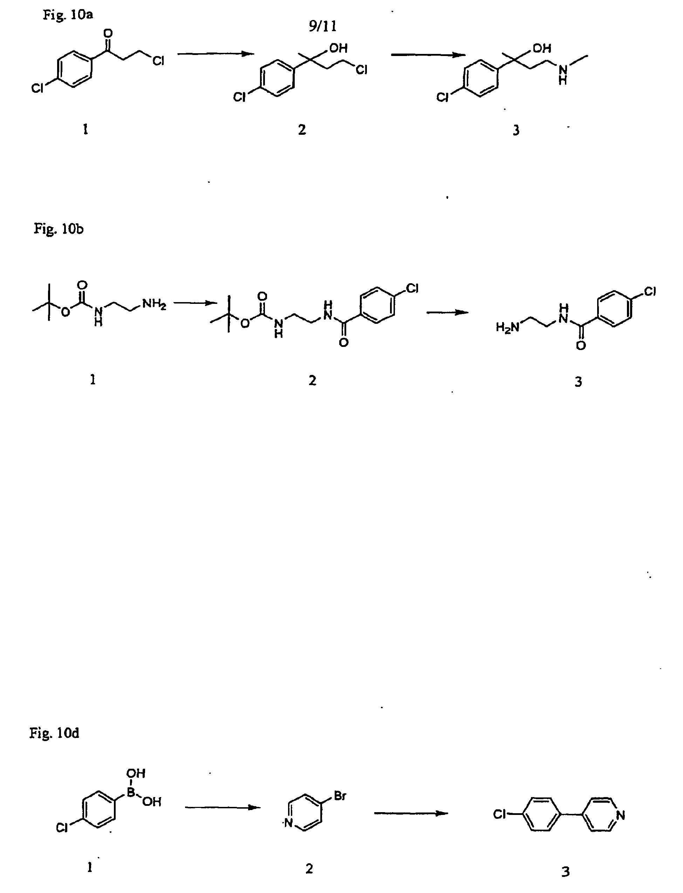

Figure 10A is a schematic showing the preparation of 3-(4-chlorophenyl)-3-hydroxyl-3-methyl-1-*N*-methylaminopropane.
Figure 10B is a schematic showing the preparation of 1-(4-chlorobenzoyl)-1,3-propylenediamine.
Figure 10D is a schematic showing the preparation of 4-(4-chlorophenyl)-4-pyridine.

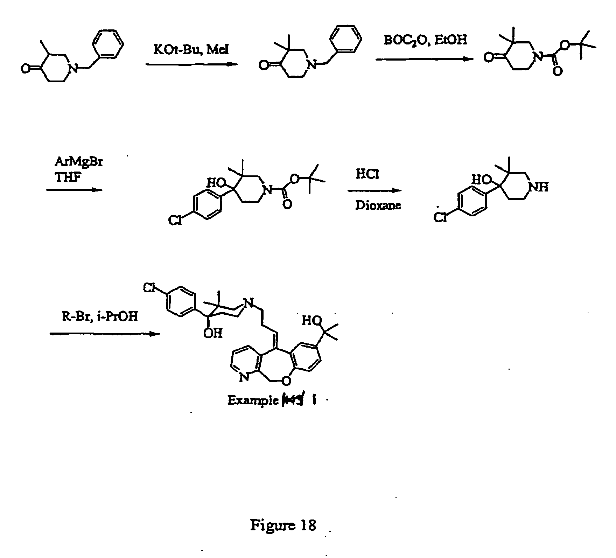

Figure 18 is a schematic showing a procedure for the preparation of Example I.

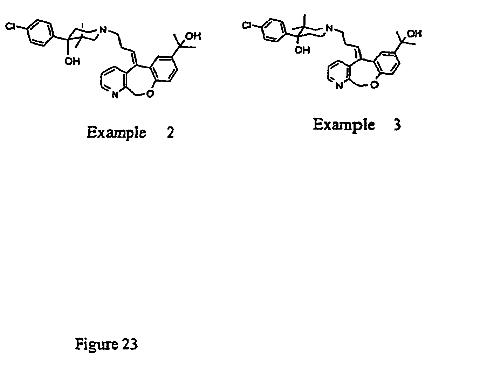

Figure 23 shows the structures of exemplary compounds of the invention.

### DETAILED DESCRIPTION OF THE INVENTION

The present invention relates to small molecule compounds which are modulators of chemokine receptor function. In a preferred embodiment, the small molecule compounds are antagonists of chemokine receptor function. Accordingly, processes or cellular responses mediated by the binding of a chemokine to a receptor can be inhibited (reduced or prevented, in whole or in part), including leukocyte migration, integrin activation, transient increases in the concentration of intracellular free calcium [Ca⁺⁺]ᵢ, and/or granule release of proinflammatory mediators.

The invention further relates to the treatment, including prophylactic and therapeutic treatments, of a disease associated with aberrant leukocyte recruitment and/or activation or mediated by chemokines or chemokine receptor function, including chronic inflammatory disorders characterized by the presence of RANTES, MIP-1α, MCP-2, MCP-3 and/or MCP-4 responsive T cells, monocytes and/or eosinophils, including but not limited to diseases such as arthritis (e.g., rheumatoid arthritis), atherosclerosis, arteriosclerosis, restenosis, ischemia/reperfusion injury, diabetes mellitus (e.g., type 1 diabetes mellitus), psoriasis, multiple sclerosis, inflammatory bowel diseases such as ulcerative colitis and Crohn's disease, rejection of transplanted organs and tissues (i.e., acute allograft rejection, chronic allograft rejection), graft versus host disease, as well as allergies and asthma. Other diseases associated with aberrant leukocyte recruitment and/or activation which can be treated (including prophylactic treatments) with the methods disclosed herein are inflammatory diseases associated with Human Immunodeficiency Virus (HIV) infection, e.g., AIDS associated encephalitis, AIDS related maculopapular skin eruption, AIDS related interstitial pneumonia, AIDS related enteropathy, AIDS related periportal hepatic inflammation and AIDS related glomerulo nephritis. The treatment comprises administering to the subject in need of treatment an effective amount of a compound (i.e., one or more compounds) which inhibits chemokine receptor function, inhibits the binding of a chemokine to leukocytes and/or other cell types, and/or which inhibits leukocyte migration to, and/or activation at, sites of inflammation.

The invention further relates to methods of antagonizing a chemokine receptor, such as CCR1, in a mammal comprising administering to the mammal a compound as described herein.

According to the method, chemokine-mediated chemotaxis and/or activation of pro-inflammatory cells bearing receptors for chemokines can be inhibited. As used herein, "pro-inflammatory cells" includes but is not limited to leukocytes, since chemokine receptors can be expressed on other cell types, such as neurons and epithelial cells.

While not wishing to be bound by any particular theory or mechanism, it is believed that compounds of the invention are antagonists of the chemokine receptor CCR1, and that therapeutic benefits derived from the method of the invention are the result of antagonism of CCR1 function. Thus, the method and compounds of the invention can be used to treat a medical condition involving cells which express CCR1 on their surface and which respond to signals transduced through CCR1, as well as the specific conditions recited above.

In one embodiment, the antagonist of chemokine receptor function is represented by Structural Formula (I): 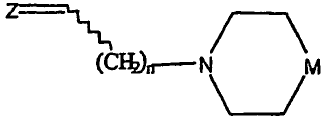 and physiologically acceptable salts thereof.

In such compounds:
n is two;
M is >C(OH)R²;
R² is 4-chlorophenyl;
the ring containing M is substituted; and
Z is a tricyclic ring system provided by Structural Formula (III) 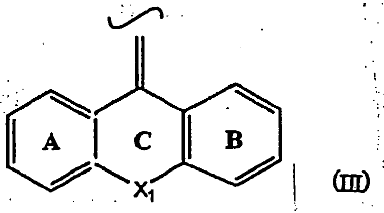 wherein X₁ is -CH₂-O-, Ring A is a pyridyl group, Ring B is a phenyl group and Ring B is substituted para to the carbon atom of Ring B which is bonded to X₁ of Ring C.

Thus Z can be represented by Structural Formula (VI): 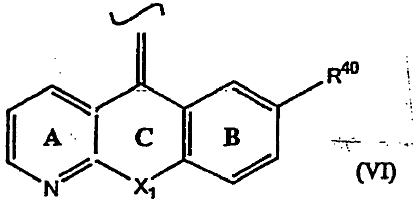 wherein X is -CH₂-O- and R⁴⁰ is

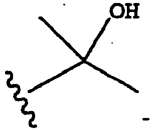

In this regard, the compounds of the invention are further represented by formula: 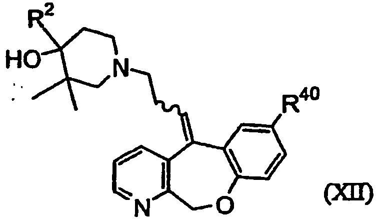 or a physiological acceptable salt thereof, wherein R² is 4-chlorophenyl and R⁴⁰ is as described herein.

In a particularly preferred embodiment, the compound is the (S)-enantiomer of the compound of Formula (XII) and has the structure: 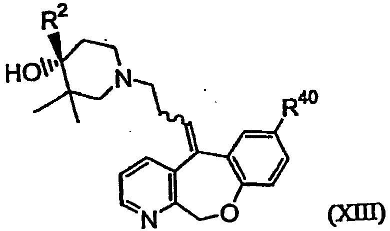 or a physiologically acceptable salt thereof, wherein R² is 4-chlorophenyl and R⁴⁰ is

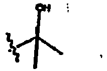

The compounds disclosed herein can be obtained as E- and Z-configurational isomers. It is expressly pointed out that the invention includes compounds of the E-configuration and the Z-configuration around the double bond connecting Ring C of Z to the remainder of the molecule, and a method of treating a subject with compounds of the E-configuration, the Z-configuration, and mixtures thereof. Accordingly, in the structural formulas presented herein, the symbol: 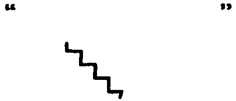 is used to represent both the E configuration and the Z configuration. Preferably Ring A and the alkylene chain bonded to Ring C are in the cis configuration. For example, the compounds can have the configuration of:

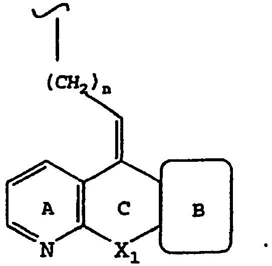

It is understood that one configuration can have greater activity than another. The desired configuration can be determined by screening for activity, employing the methods described herein.

Additionally, certain compounds of the invention may be obtained as different stereoisomers (e.g., diastereomers and enantiomers). The compounds of the invention can be prepared as racemates or as substantially pure stereoisomers. The stereoisomers of the invention (e.g., (*S*)- and (*R*)-enantiomers) can be prepared using any suitable method. For example, the enantiomers can be resolved from the racemate using chiral chromatography or recrystallization. Preferably, the stereoisomers (e.g., (*S*)- and/or (*R*)-enantiomers) are prepared by stereospecific synthesis as described herein.

The optical configuration of the stereoisomers of the invention are assigned using the (*R*),(*S*) method of Cahn-Ingold-Prelog. (See, J. March, "Advanced Organic Chemistry," 4th Edition, Wiley Interscience, New York, pp.109-111 (1992).)

The invention includes all isomeric forms and racemic mixtures of the disclosed compounds and a method of treating a subject with both pure isomers and mixtures thereof, including racemic mixtures. Sterioisomers can be separated and isolated using any suitable method, such as chromatography. Again, it is understood that one sterioisomer may be more active than another. The desired isomer determined by screening.

Also included in the present invention are physiologically acceptable salts of the compounds described herein. Salts of compounds containing an amine or other basic group can be obtained, for example, by reacting with a suitable organic or inorganic acid, such as hydrogen chloride, hydrogen bromide, acetic acid, citric acid, perchloric acid and the like. Compounds with a quaternary ammonium group also contain a counteranion such as chloride, bromide, iodide, acetate, perchlorate and the like. Salts of compounds containing a carboxylic acid or other acidic functional group can be prepared by reacting with a suitable base, for example, a hydroxide base. Salts of acidic functional groups contain a countercation such as sodium, potassium, ammonium, calcium and the like. (See, for example, Berge S.M. et al., "Pharmaceutical Salts," J. Pharma. Sci., 66:1 (1977).)

In the structural formulas depicted herein, the single or double bond by which a chemical group or moiety is connected to the remainder of the molecule or compound is indicated by the following symbol: 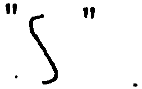 For example, the corresponding symbol in Structural Formula (III) indicates the double bond by which the central ring of the tricyclic ring system is connected to the remainder of the molecule represented by Structural Formula (I).

A "subject" is preferably a bird or mammal, such as a human, but can also be an animal in need of veterinary treatment, e.g., domestic animals (e.g., dogs, cats, and the like), farm animals (e.g., cows, sheep, fowl, pigs, horses, and the like) and laboratory animals (e.g., rats, mice, guinea pigs, and the like).

An "effective amount" of a compound is an amount which results in the inhibition of one or more processes mediated by the binding of a chemokine to a receptor in a subject with a disease associated with aberrant leukocyte recruitment and/or activation. Examples of such processes include leukocyte migration, integrin activation, transient increases in the concentration of intracellular free calcium [Ca²⁺]ᵢ and granule release of proinflammatory mediators. Alternatively, an "effective amount" of a compound is a quantity sufficient to achieve a desired therapeutic and/or prophylactic effect, such as an amount which results in the prevention of or a decrease in the symptoms associated with a disease associated with aberrant leukocyte recruitment and/or activation.

The amount of compound administered to the individual will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. Typically, an effective amount of the compound can range from about 0.1 mg per day to about 100 mg per day for an adult. Preferably, the dosage ranges from about 1 mg per day to about 100 mg per day. An antagonist of chemokine receptor function can also be administered in combination with one or more additional therapeutic agents, e.g. theophylline, β-adrenergic bronchodilators, corticosteroids, antihistamines, antiallergic agents, immunosuppressive agents (e.g., cyclosporin A, FK-506, prednisone, methylprednisolone), hormones (*e.g*., adrenocorticotropic hormone (ACTH)), cytokines (*e.g*., interferons (*e.g*., IFNβ-1a, IFNβ-1b)) and the like.

The compound can be administered by any suitable route, including, for example, orally in capsules, suspensions or tablets or by parenteral administration. Parenteral administration can include, for example, systemic administration, such as by intramuscular, intravenous, subcutaneous, or intraperitoneal injection. The compound can also be administered orally (e.g., dietary), transdermally, topically, by inhalation (e.g., intrabronchial, intranasal, oral inhalation or intranasal drops), or rectally, depending on the disease or condition to be treated. Oral or parenteral administration are preferred modes of administration.

The compound can be administered to the individual in conjunction with an acceptable pharmaceutical or physiological carrier as part of a pharmaceutical composition for treatment of HIV infection, inflammatory disease, or the other diseases discussed above. Formulation of a compound to be administered will vary according to the route of administration selected (e.g., solution, emulsion, capsule). Suitable carriers may contain inert ingredients which do not interact with the compound. Standard pharmaceutical formulation techniques can be employed, such as those described in Remington's Pharmaceutical Sciences, Mack Publishing Company, Easton, PA. Suitable carriers for parenteral administration include, for example, sterile water, physiological saline, bacteriostatic saline (saline containing about 0.9% benzyl alcohol), phosphate-buffered saline, Hank's solution, Ringer's-lactate and the like. Methods for encapsulating compositions (such as in a coating of hard gelatin or cyclodextran) are known in the art (Baker, et al., "Controlled Release of Biological Active Agents", John Wiley and Sons, 1986).

The quantity of active ingredient (one or more compounds of the invention) in the composition can range from about 0.1% to about 99.9% by weight. Preferably the quantity of active ingredient is about 10% to about 90%, or about 20% to about 80% by weight. A unit dose preparation can contain from 1 mg to about 1000 mg active ingredient, preferably about 10 mg to about 100 mg active ingredient. The composition can, if desired, also contain other compatible therapeutic agents, such as theophylline, β-adrenergic bronchodilators, corticosteroids, antihistamines, antiallergic agents, immunosuppressive agents (*e.g*., cyclosporin A, FK-506, prednisone, methylprednisolone), hormones (*e.g*., adrenocorticotropic hormone (ACTH)), cytokines (*e.g*., interferons (*e.g*., IFNβ-1a, IFNβ-1b)) and the like.

In one embodiment, the pharmaceutical composition comprises the (*S*)-enantiomer of a compound of the invention (*e.g*., a compound of Structural Formula (XIII) and a physiologically acceptable carrier or excipient. For example, in one embodiment, the composition comprises *(S)*-4-(4-Chloro-phenyl)-1-{3-[7-(1-hydroxy-1-methyl-ethyl)-11H-10-oxa-1-aza-dibenzo[a,d]cyclohepten-5-ylidene]-propyl}-3,3-dimethyl-piperidin-4-ol and a physiologically acceptable carrier or excipient. In certain embodiments, the pharmaceutical composition comprises the (*S*)-enantiomer of a compound of the invention (*e.g*., a compound of Structural Formula (XIII) and is substantially free of the corresponding (*R*)-enantiomer (contains at least about 98% or at least about 99% enantiomeric excess of (*S*)-enantiomer). In another embodiments, the composition comprises the (*S*)-enantiomer of a compound of the invention (e.g., a compound of Structural Formula (XII)), the corresponding (R)-enantiomer and a physiologically acceptable carrier or excipient. In a more particular embodiment, the composition comprises a racemic compound of Structural Formula (XII), for example, racemic-4-(4-Chloro-phenyl)-1-{3-[7-(1-hydroxy-1-methyl-ethyl)-11H-10-oxa-1-aza-dibenzo[a,d]cyclohepten-5-ylidene]-propyl}-3,3-dimethyl-piperidin-4-ol In other embodiments, the ratio (*S*)-enantiomer:(*R*)-enantiomer (w/w) in the compositions is at least about 2:1 or about 5:1 or about 10:1 or about 20:1 or about 50:1.

The activity of compounds of the present invention can be assessed using suitable assays, such as receptor binding assays and chemotaxis assays. For example, as described in the Exemplification Section, small molecule antagonists of RANTES and MIP-1α binding have been identified utilizing THP-1 cells which bind RANTES and chemotax in response to RANTES and MIP-1α as a model for leukocyte chemotaxis. Specifically, a high through-put receptor binding assay, which monitors ¹²⁵I-RANTES and ¹²⁵I-MIP-1α binding to THP-1 cell membranes, was used to identify small molecule antagonists which block binding of RANTES and MIP-1α. Compounds of the present invention can also be identified by virtue of their ability to inhibit the activation steps triggered by binding of a chemokine to its receptor, such as chemotaxis, integrin activation and granule mediator release. They can also be identified by virtue of their ability to block RANTES and MIP-1α mediated HL-60, T-cell, peripheral blood mononuclear cell, and eosinophil chemotactic response.

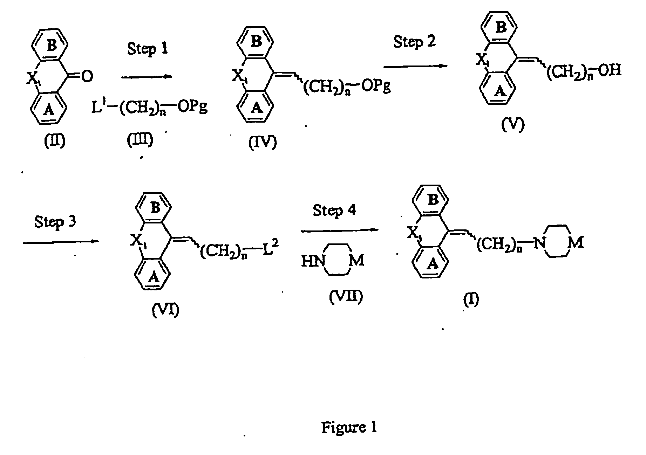

The compounds disclosed herein can be prepared accordingly to the schemes shown in Figures 1-4 and 7. The schemes are described in greater detail below.

Figure 1 shows the preparation of compounds represented by Structural Formula (I). L¹ is PPh₃Cl, PPh₃Br, PPh₃I or (EtO)₂P(O), L² is a suitable leaving group such as halogen, p-toluene sulfonate, mesylate, alkoxy, and phenoxy; Pg is a suitable protecting group such as tetrahydropyranyl; and the other symbols are as defined above.

In Step 1 of Figure 1, a Wittig reaction is carried out in a solvent such as ether, or tetrahydrofuran (THF) in the presence of a base such as sodium hydride, n-butyl lithium or lithium diisopropylamide (LDA) at 0°C up to the reflux temperature for the solvent used for 5 minutes to 72 h. Compounds represented by Formula II in Figure 1 can be prepared by methods disclosed in JP 61/152673, U.S. Patent 5089496, WO 89/10369, WO 92/20681 and WO 93/02081, the entire teachings of which are incorporated herein by reference.

In Step 2 of Figure 1, deprotection is carried out with an acid in a solvent such as methanol at room temperature up to the reflux temperature for the solvent used for 5 minutes to 72 h. Alternatively, a compound of represented by Formula V in Figure 1 can be prepared directly from step 1 without isolating an intermediate. The reaction mixture obtained after the work up of the reaction described in step 1 can be dissolved in the solvent and reacted with the acid.

In Step 3 of Figure 1, the hydroxy group can be converted to a leaving group by known methods. Compounds represented by Formula VI in Figure 1 can be prepared by methods disclosed in J. Med. Chem., 1992 (35) 2074-2084 and JP 61/152673.

In Step 4 of Figure 1, an alkylation reaction is carried out in a solvent such as acetone, methyl ethyl ketone, ethyl acetate, toluene, tetrahydrofuran (THF) or dimethylformamide (DMF) in the presence of a base such as potassium carbonate or sodium hydride and a catalyst such as an alkali metal iodide at room temperature up to the reflux temperature for the solvent used for 5 minutes to 72 h.

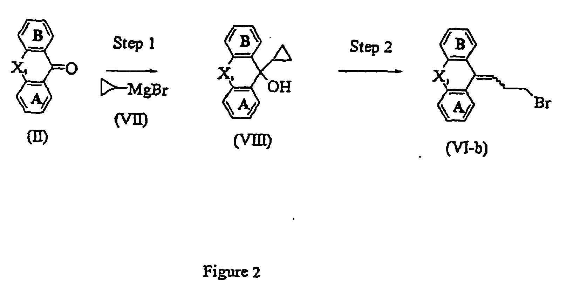

Figure 2 shows the preparation of compounds represented by Compound (VI-b). In Step 1 of Figure 2, a Grignard reaction may be carried out in a solvent such as ether, or tetrahydrofuran (THF) at 0°C up to the reflux temperature for the solvent used for 5 minuets to 72 h. Compound VII is available commercially.

In Step 2 of Figure 2, bromination may be carried out with brominate agents such as hydrobromic acid, bromotrimethylsilane or boron tribromide-methyl sulfide complex in a solvent such as acetic acid, dichloromethane or dichloroethane at room temperature up to the reflux temperature for the solvent used for 5 minutes to 72 h.

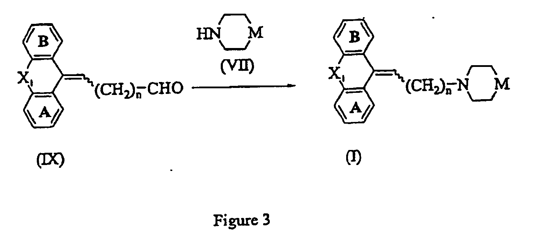

Figure 3 shows the preparation of compounds represented by Structural Formula (I). In Figure 3, a reductive amination may be carried out with reducing regents such as sodium cyanoborohydride, sodium acetoxyborohydride or sodium borohydride in a solvent such as methanol, ethanol, tetrahydrofuran (THF), dichloromethane or dichloroethane at room temperature up to the reflux temperature for the solvent used for 5 minutes to 72 h.

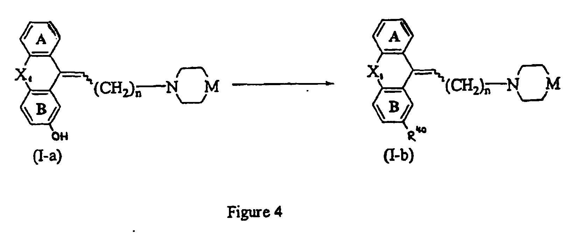

Figure 4 shows the preparation of compounds represented by Structural Formula (I), where in Z is represented by Structural Formulas (III) and wherein Ring B in Z is substituted with R⁴⁰. In Figure 4, the alkylation reaction can be carried out in a solvent such as acetone, methyl ethyl ketone, ethyl acetate, toluene, tetrahydrofuran (THF) or dimethylformamide (DMF) in the presence of a base such as potassium carbonate or sodium hydride and a catalyst such as an alkali metal iodide at room temperature up to the reflux temperature for the solvent used for 5 minutes to 72 h.

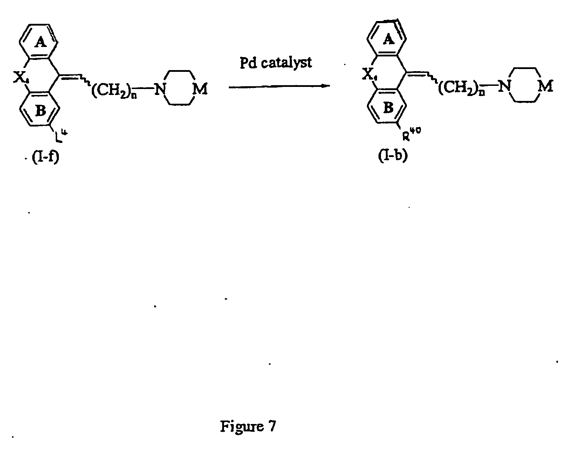

Figure 7 shows the preparation of compounds represented by Structural Formula (I), wherein Z is represented by Structural Formulas (III) and wherein Ring B in Z is substituted with R⁴⁰. L⁴ is a suitable leaving group such as halogen or trifluoromethylsulfonate. In Figure 7, a palladium coupling reaction such as Stille coupling, Suzuki coupling, Heck reaction, or carboxylation using carbon monoxide maybe carried out using a palladium catalyst such as tetrakis(triphenylphosphine)palladium, bis(triphenylphosphine)palladium chloride, and palladium acetate in a solvent such as tetrahydrofuran (THF), 1,4-dioxane, toluene, dimethylformamide (DMF), or dimethylsufoxide (DMSO) in the presence of additive (when necessary) such as triphenylphosphine, 1,1'-bis(diphenylphosphino)ferrocene, triethylamine, sodium bicarbonate, tetraethylammonium chloride, or lithium chloride at room temperature up to the reflux temperature for the solvent used for 5 minutes to 72 h.

Although Figures 1-4 and 7 show the preparation of compounds in which Rings A and B are phenyl rings, analogous compounds with heteroaryl groups for Rings A and B can be prepared by using starting materials with heteroaryl groups in the corresponding positions. These starting materials can be prepared according to methods disclosed in JP 61/152673, U.S. Patent 5089496, WO 89/10369, WO 92/20681 and WO 93/02081.

The invention is illustrated by the following examples which are not intended to be limiting in any way.

### EXEMPLIFICATION

### Example 1:

4-(4-Chloro-phenyl)-1-{3-[7-(1-hydroxy-1-methyl-ethyl)-11H-10-oxa-1-aza-dibenzo[a,d]cyclohepten-5-ylidene]-propyl}-3,3-dimethyl-piperidin-4-ol

### Step 1: 1-Benzyl-3,3-dimethyl-piperidin-4-one

To a solution of 1-Benzyl-3-methyl-piperidin-4-one (2.03 g, 10 mmol) in THF (10 mL) was added potassium t-Butoxide (1.1g, 10 mmol) and methyl iodide ( 0.62 mL, 10 mmol). The solution was then stirred at room temperature for about 72 hours. The reaction was quenched with brine and extracted with ethyl acetate. The combined organic layers were concentrated *in vacuo,* then purified by flash chromatography on silica gel (35g SiO₂, gradient elution from 100% hexane to 100% ethyl acetate) to yield the title compound as a colorless oil. ¹H-NMR (CDCL₃) ∂: 1.12 (6H, s), 2.41 (2H, s), 2.52 (2H, m), 2.73 (2H, m), 3.56 (2H, s), 7.20-7.40 (5H, m). ESI-MS m/z: 218 (M + 1).

### Step 2: 3,3-Dimethyl-4-oxo-piperidine-1-carboxylic acid tert-butyl ester

To a solution of 1-Benzyl-3,3-dimethyl-piperidin-4-one (2.48 g, 11 mmol) in ethanol (100 mL) was added di-tert-butyl dicarbonate (2.18, 10 mmol) and palladium hydroxide (0.10 g), The suspension was then shaken under a hydrogen atmosphere (40 PSI) at room temperature for about an additional 12 hours. The reaction was filtered through celite and evaporated *in vacuo* to yield the title compound as a white solid.
¹H-NMR (CDCL₃) ∂: 1.12 (6H, br s), 1.48 (9H, s), 2.45-2.80 (3H, m), 3.12 (1H, m), 3.42 (1H, br s), 3.70 (1H, m).

### Step 3: 4-(4-Chloro-phenyl)-4-hydroxy-3,3-dimethyl-piperidine-1-carboxylic acid tert-butyl ester

To an ice-cooled solution 3,3-Dimethyl-4-oxo-piperidine-1-carboxylic acid tert-butyl ester (1.6 g, 7.2 mmol) in THF (20 mL) was added 4-chlorophenylmagnesium bromide (1 M in ether, 15 mL, 15 mmol). The solution was allowed to warm to about room temperature, then stirred at room temperature for about 22 hours. The reaction was quenched with aqueous ammonium chloride and extracted with ethyl acetate. The combined organic layers were concentrated *in vacuo*, then purified by flash chromatography on silica gel (35g SiO₂, gradient elution from 100% hexane to 100% ethyl acetate) to yield the title compound as a colorless oil.
¹H-NMR (CDCL₃) ∂: 1.12 (6H, s), 1.50 (11H, m), 2.60 (1H, m), 3.20 (2H, m), 3.56 (1H, m), 4.20 (1H, m), 7.20-7.40 (4H, m). ESI-MS m/z: 218 (M + 1).

### Step 4: 4-(4-Chloro-phenyl)-3,3-dimethyl-piperidin-4-ol

4-(4-Chloro-phenyl)-4-hydroxy-3,3-dimethyl-piperidine-1-carboxylic acid tert-butyl ester (0.25 g, 0.73 mmol) was dissolved in 4M HCl/Dioxane (2 mL, 8 mmol). The solution was stirred at room temperature for about 4 hours. The solvent was removed *in vacuo.* The residue was quenched with aqueous sodium hydroxide, extracted with ethyl acetate, and the organic layers were dried over sodium sulfate and evaporated *in vacuo* to yield the title compound as a yellow solid. The mixture was carried on to the next step without further purification.

### 4-(4-Chloro-phenyl)-1-{3-[7-(1-hydroxy-1-methyl-ethyl)-11H-10-oxa-1-aza-dibenzo[a,d]cyclohepten-5-ylidene]-propyl}-3,3-dimethyl-piperidin-4-ol

To a solution of the amino alcohol mixture (0.17 g, 0.7mmol) in isopropanol (5 mL) was added 2,6-lutidine (0.23 mL, 2.0 mmol) and catalytic potassium iodide. This mixture was heated to about 80°C, and treated with 2-[5-(3-Bromo-propylidene)-5,11-dihydro-10-oxa-1-aza-dibenzo[a,d]cyclohepten-7-yl]-propan-2-ol (0.19 g, 0.5 mmol), added in portions over about 2 hours. The solution was then stirred at about 80°C for an additional 2 hours. The reaction was concentrated *in vacuo,* then purified by flash chromatography on silica gel (10g SiO₂, gradient elution from 100% ethyl acetate to 87% ethyl acetate:10% methanol:3% triethylamine) to yield the title compound as a brown semisolid. ¹H-NMR (CDCL₃) ∂: 0.78 (3H, s), 0.90 (3H, s), 1.45 (2H, m), 1.53 (6H, s), 2.28-2.80 (9H, m), 5.30 (2H, br s), 6.15 (1H, t, J = 1.4 Hz), 6.79 (2H, d, J = 8.4 Hz), 7.18-7.45 (7H, m), 7.59 (1H, d, J = 8 Hz), 8.45 (1H, m). ESI-MS m/z: 533 (M + 1).

### Example 2

### S-4-(4-Chloro-phenyl)-1-{3-[7-(1-hydroxy-1-methyl-ethyl)-11H-10-oxa-1-aza-dibenzo[a,d]cyclohepten-5-ylidene]-propyl}-3,3-dimethyl-piperidin-4-ol

### Step 1

To a dry, 2L 2-neck, round-bottom flask equipped with a magnetic stirrer, a condenser, and a large 10 °C water bath was added 4-oxo-piperidine-1-carboxylic acid tert-butyl ester (125 g, 628 mmol) and anhydrous tetrahydrofuran (1 L). To the resulting yellow solution was added methyl iodide (85 mL, 1365 mmol). Sodium t-butoxide (150g,1560 mmol) was then added portionwise over 30 minutes. An exotherm was detected, especially at the beginning of the addition. The reaction mixture did warm to a gentle reflux, the rate was controlled by the speed of addition of base. The mixture was stirred an additional 30 minutes. The solvent was removed *in vacuo*. The oily residue was treated with NH₄Cl/water (500 mL), and extracted with ether (3 x 200 mL). The combined organics were washed with brine, dried over Na₂SO₄, and filtered through a short plug of silica gel. The solvent was removed *in vacuo*, and the resulting yellow oil had started to ccrystallize. It was left under high vacuum overnight. The mixture was slurried in hexane (50-100 mL) and sonicated for one minute. The yellow solid was collected by filtration and washed with hexane (100 mL). The first crop of 3,3-dimethyl-4-oxo-piperidine-1-carboxylic acid tert-butyl ester yielded a yellow solid. (See, preparation of (37) in Vice, S. et al., J. Org. Chem., 66:2487-2492 (2001).)
¹H-NMR (CDCl₃, 300 MHz) ∂: 1.13 (s, 6 H), 1.49 (s, 9 H), 2.49 (t, 2 H), 3.43 (br s, 2 H), 3.73 (t, 2 H).

### Step 2

A 2-neck, 2-L round bottom flask was fitted with 2 125 mL dropping funnels and a stir bar. The assembly was flame-dried under dry nitrogen. The flask was charged with THF (700 mL) and 4-bromo-chlorobenzene (33.7 g, 176 mmol, 2.5 eq.). The resulting solution was cooled to -78 °C in a dry ice/acetone bath. To one of the dropping funnels was added butyllithium (2.5 M in hexanes, 70 mL, 175 mmol, 2.5 eq) via canula. The butyllithium solution was slowly added to the cold THF solution over 1 h. Stirring continued for an additional 0.5 h affording a white suspension. A solution of 3,3-dimethyl-4-oxo-piperidine-1-carboxylic acid tert-butyl ester (16.0 g, 70.5 mmol, 1 eq.) in THF (100 mL) was prepared and added to the reaction mixture via the second dropping funnel over 1.75 h. The resulting mixture was stirred at -78 °C for 2h, at which time reaction appears to be essentially complete by TLC analysis. Saturated aqueous NH₄Cl (150 mL) was added and the reaction was allowed to warm to rt. Water (150 mL) was added and the mixture extracted with ethyl acetate (2 + 1L). The combined extracts were washed with water and brine, dried over magnesium sulfate, filtered and concentrated. The solid residue was triturated with ethyl acetate and filtered. The supernatant was concentrated and triturated with ether. The resulting supernatant was then triturated with ether/petroleum ether. The resulting solids were combined to afford 4-(4-Chloro-phenyl)-4-hydroxy-3,3-dimethyl-piperidine-1-carboxylic acid tert-butyl ester (17.52 g, 51.6 mmol, 73 %) as an off-white solid.
¹H-NMR (CDCl₃, 300 MHz) δ: 0.82 (s, 6 H), 1.34 -1.44 (m, 2H), 1.49 (s, 9 H), 2.67 (ddd, 1 H), 3.10 - 3.70 (m, 3 H), 4.00 - 4.30 (m, 1H), 7.31 (d, 2 H), 7.39 (d, 2 H).

### Step 3

To a cooled (0 °C) solution of the compound prepared in step 2 (10.42 g, 30.7 mmol) in methylene chloride (300 mL) was slowly added trifluoroacetic acid (60 mL) over 1.25 h. The resulting yellow solution was stirred at 0 °C for an additional 1.5 h. The mixture was concentrated under reduced pressure and the residue dissolved in ethyl acetate (1.2 L), washed with aqueous sodium hydroxide (1 N, 150 mL). The aqueous layer was extracted with additional ethyl acetate (200 mL) and the combined extracts were washed with brine, dried over sodium sulfate, filtered and concentrated. The resulting solid residue was triturated with ether to afford 4-(4-chloro-phenyl)-3,3-dimethyl-piperidin-4-ol (6.94 g, 29.0 mmol, 94 %) as an off-white solid.
¹H-NMR (CD₃OD, 300 MHz) δ: 0.73 (s, 3 H), 0.85 (s, 3 H), 1.42 (ddd, 1 H), 2.36 (d, 1 H), 2.61 (ddd, 1 H), 2.91 (br dd, 1 H), 3.08 - 3.19 (m, 2 H), 7.26 - 7.32 (m, 2 H), 7.44 - 7.50 (m, 2 H).
MS m/z: 240 (M + 1).

### Step 4

A visibly clean 5 L, 3-neck flask was fitted with an overhead stirrer and flushed with nitrogen for 20 min. 4-(4-Chloro-phenyl)-3,3-dimethyl-piperidin-4-ol (202g, 843 mmol), L-(+)-tartaric acid (114 g, 759 mmol) and 4040 mL of a 9:1 butanone:water mixture were added to the flask. The mixture was heated to reflux. Water (202 mL) was added portionwise over 45 min (ratio of butanone to water: 6:1) to fully dissolve the solid mixture. Reflux was continued an additional 45 min, the heat source was then turned off and the flask allowed to cool slowly to rt - overnight. Solids were removed under suction filtration and dried 3 d in *vacuo* to afford S-enantiomer (134.4 g, 41 %) as the L-(+) tartrate salt.
The above salt was partitioned between 1 M NaOH and methylene chloride (brine washed and sodium sulfate-dried) to afford the free base. ¹H-NMR (CD₃OD, 300 MHz) δ: 0.73 (s, 3 H), 0.85 (s, 3 H), 1.42 (ddd, 1 H), 2.36 (d, 1 H), 2.61 (ddd, 1 H), 2.91 (br dd, 1 H), 3.08 - 3.19 (m, 2 H), 7.26 = 7.32 (m, 2 H), 7.44 - 7.50 (m, 2 H).
MS m/z: 240 (M + 1).

### Step 5

To a solution of the homochiral product of step 4 (2.40 g, 10 mmol) in acetonitrile (80 mL) and water (20 mL) was added potassium carbonate (1.39 g, 10 mmol) followed by 2-[5-(3-bromo-propylidene)-5,11-dihydro-10-oxa-1-aza-dibenzo[a,d]cyclohepten-7-yl]-propan-2-ol (2.49 g, 6.67 mmol). The biphasic mixture was stirred at rt 48 h. Acetonitrile was removed by rotary evaporation and the resulting slurry was partitioned between ethyl acetate and brine. The organic phase was dried over magnesium sulfate, filtered and concentrated to afford an oily solid which was purified by silica gel chromatography (methylene chloride - 90/10 methylene chloride/methanol gradient) to afford the title compound as an off-white powder (2.63 g, 74 %).
¹H-NMR (CD₃OD, 300 MHz) δ: 0.71 (s, 3 H), 0.84 (s, 3 H), 1.4-1.55 (m, 9 H), 2.18 - 2.81 (m, 9 H), 5.15 - 5.40 (br s, 2 H), 6.23 (t, 1 H), 6.74 (d, 1 H), 7.23 - 7.31 (m, 3 H), 7.42 - 7.50 (m, 4 H), 7.80 (dd, 1 H), 8.47 (dd, 1 H). MS m/z: 533 (M + 1).

### Example 3

### R-4-(4-Chloro-phenyl)-1-{3-[7-(1-hydroxy-1-methyl-ethyl)-11H-10-oxa-1-aza-dibenzo[a,d]cyclohepten-5-ylidene]-propyl}-3,3-dimethyl-piperidin-4-ol

### Step 1

Racemic 4-(4-chloro-phenyl)-3,3-dimethyl-piperidin-4-ol (0.500 g, 2.086 mmol) was dissolved in minimal hot isopropyl alcohol (ca. 5 mL). The hot solution was filtered through a plug of cotton and transferred to a solution of (1S)-(+)-10-camphorsulfonic acid (0.484 g, 2.086 mmol) in isopropyl alcohol (ca. 3 mL). The mixture was stirred vigorously for several minutes, during which a thick precipitate forms, and allowed to cool to rt over 0.25 h. The solids were removed by suction filtration and dried *in vacuo.* The dried salt was dissolved in hot isopropyl alcohol (ca. 50 mL), filtered thorugh a cotton plug, and allowed to slowly cool to rt, undisturbed, overnight. The solids that formed on cooling (95 mg, 19 % of theoretical) were removed by suction filtration and shown by analytical HPLC to be enantiomerically pure. The salt was suspended in ethyl acetate and neutralized with sodium hydroxide (1 N). The homogenous organic phase was washed with water and brine, dried over sodium sulfate, filtered and dried to afford R-4-(4-chloro-phenyl)-3,3-dimethyl-piperidin-4-ol.
¹H-NMR (CD₃OD, 300 MHz) δ: 0.73 (s, 3 H), 0.85 (s, 3 H), 1.42 (ddd, 1H), 2.36 (d, 1 H), 2.61 (ddd, 1 H), 2.91 (br dd, 1 H), 3.08 - 3.19 (m, 2 H), 7.26 - 7.32 (m, 2 H), 7.44 - 7.50 (m, 2 H).
MS m/z: 240 (M + 1).

### Step 2

The titled compound was prepared following the procedure in step 5 of Example 502, substituting S-4-(4-chloro-phenyl)-3,3-dimethyl-piperidin-4-ol for R-4-(4-chloro-phenyl)-3,3-dimethyl-piperidin-4-ol.
¹H-NMR (CD₃OD, 300 MHz) δ: 0.71 (s, 3 H), 0.84 (s, 3 H), 1.4 - 1.55 (m, 9 H), 2.18 - 2.81 (m, 9 H), 5.15 - 5.40 (br s, 2 H), 6.23 (t, 1 H), 6.74 (d, I H), 7.23 - 7.31 (m, 3 H), 7.42 - 7.50 (m, 4 H), 7.80 (dd, I H), 8.47 (dd, I H). MS m/z: 533 (M + 1).

### Example 4: Membrane Preparations for Chemokine Binding and Binding Assays

Membranes were prepared from THP-1 cells (ATCC #TTB202). Cells were harvested by centrifugation, washed twice with PBS (phosphate-buffered saline), and the cell pellets were frozen at -70 to -85°C. The frozen pellet was thawed in ice-cold lysis buffer consisting of 5 mM HEPES (N-2-hydroxyethylpiperazine-N-2-ethane-sulfonic acid) pH 7.5, 2 mM EDTA (ethylenediaminetetraacetic acid), 5 µg/ml each aprotinin, leupeptin, and chymostatin (protease inhibitors), and 100 µg/ml PMSF (phenyl methane sulfonyl fluoride - also a protease inhibitor), at a concentration of 1 to 5 x 10⁷ cells/ml. This procedure results in cell lysis. The suspension was mixed well to resuspend all of the frozen cell pellet. Nuclei and cell debris were removed by centrifugation of 400 x g for 10 minutes at 4°C. The supernatant was transferred to a fresh tube and the membrane fragments were collected by centrifugation at 25,000 x g for 30 minutes at 4°C. The supernatant was aspirated and the pellet was resuspended in freezing buffer consisting of 10 mM HEPES pH 7.5, 300 mM sucrose, 1µg/ml each aprotinin, leupeptin, and chymostatin, and 10 µg/ml PMSF (approximately 0.1 ml per each 10⁸ cells). All clumps were resolved using a minihomogenizer, and the total protein concentration was determined using a protein assay kit (Bio-Rad, Hercules, CA, cat #500-0002). The membrane solution was then aliquoted and frozen at -70 to -85°C until needed.

Binding Assays utilized the membranes described above. Membrane protein (2 to 20 µg total membrane protein) was incubated with 0.1 to 0.2 nM ¹²⁵I-labelled RANTES or MIP-1α with or without unlabeled competitor (RANTES or MIP-1α) or various concentrations of compounds. The binding reactions were performed in 60 to 100 µl of a binding buffer consisting of 10 mM HEPES pH 7.2, 1 mM CaCl₂, 5 mM MgCl₂, and 0.5% BSA (bovine serum albumin), for 60 min at room temperature. The binding reactions were terminated by harvesting the membranes by rapid filtration through glass fiber filters (GF/B or GF/C, Packard) which were presoaked in 0.3% polyethyleneimine. The filters were rinsed with approximately 600 µl of binding buffer containing 0.5 M NaCl, dried, and the amount of bound radioactivity was determined by scintillation counting in a Topcount beta-plate counter.

The activities of test compounds are reported in the Table below as IC₅₀ values or the inhibitor concentration required for 50% inhibition of specific binding in receptor binding assays using ¹²⁵I-RANTES or ¹²⁵I-MIP-1α as ligand and THP-1 cell membranes. Specific binding is defined as the total binding minus the non-specific binding; non-specific binding is the amount of cpm still detected in the presence of excess unlabeled Rantes or MIP-1α.

**Table BIOLOGICAL DATA**

| Example | IC50 (µM) |
|---|---|
| 2 | <1 |
| 3 | <1 |

## Claims

1. A pharmaceutical composition comprising the (S)-enantiomer and the (R)-enantiomer of 4-(4-Chloro-phenyl)-1-{3-[7-(1-hydroxy-1-methyl-ethyl)-11H-10-oxa-1-aza-dibenzo[a,d]cyclohepten-5-ylidene]-propyl}-3,3-dimethyl-piperidin-4-ol, or a physiologically acceptable salt thereof, wherein the ratio of (S)-enantiomer to (R)-enantiomer (w/w) in the composition is at least about 2:1.

2. The pharmaceutical composition of claim 1, wherein the ratio of (S)-enantiomer to (R)-enantiomer (w/w) in the composition is at least about 5:1.

3. The pharmaceutical composition of claim 1, wherein the ratio of (S)-enantiomer to (R)-enantiomer (w/w) in the composition is at least about 10:1.

4. The pharmaceutical composition of claim 1, wherein the ratio of (S)-enantiomer to (R)-enantiomer (w/w) in the composition is at least about 20:1.

5. The pharmaceutical composition of claim 1, wherein the ratio of (S)-enantiomer to (R)-enantiomer (w/w) in the composition is at least about 50:1.

6. The pharmaceutical composition according to any one of claims 1 to 5 which comprises a physiologically acceptable carrier or excipient.

7. A pharmaceutical composition according to any one of claims 1 to 6 for use in treating a disease associated with aberrant leukocyte recruitment, activation or recruitment and activation, wherein said disease is selected from the group consisting of arthritis, atherosclerosis, arteriosclerosis, restenosis, ischemia/reperfusion injury, diabetes mellitus, psoriasis, multiple sclerosis, inflammatory bowel disease, rejection of a transplanted organ or tissue, graft versus host disease, allergy and asthma.

## Patentansprüche

1. Pharmazeutische Zusammensetzung, umfassend das (S)-Enantiomer und das (R)-Enantiomer von 4-(4-Chlorphenyl)-1-{3-[7-(1-hydroxy-1-methylethyl)-11H-10-oxa-1-aza-dibenzo[a,d]cyclohepten-5-yliden]propyl}3,3-dimethyl-piperidin-4-ol, oder ein physiologisch unbedenkliches Salz davon, wobei das Verhältnis von (S)-Enantiomer zu (R)-Enantiomer (Gew./Gew.) in der Zusammensetzung mindestens etwa 2:1 beträgt.

2. Die pharmazeutische Zusammensetzung nach Anspruch 1, wobei das Verhältnis von (S)-Enantiomer zu (R)-Enantiomer (Gew./Gew.) in der Zusammensetzung mindestens etwa 5:1 beträgt.

3. Die pharmazeutische Zusammensetzung nach Anspruch 1, wobei das Verhältnis von (S)-Enantiomer zu (R)-Enantiomer (Gew./Gew.) in der Zusammensetzung mindestens etwa 10:1 beträgt.

4. Die pharmazeutische Zusammensetzung nach Anspruch 1, wobei das Verhältnis von (S)-Enantiomer zu (R)-Enantiomer (Gew./Gew.) in der Zusammensetzung mindestens etwa 20:1 beträgt.

5. Die pharmazeutische Zusammensetzung nach Anspruch 1, wobei das Verhältnis von (S)-Enantiomer zu (R)-Enantiomer (Gew./Gew.) in der Zusammensetzung mindestens etwa 50:1 beträgt.

6. Die pharmazeutische Zusammensetzung nach einem der Ansprüche 1 bis 5, die einen physiologisch unbedenklichen Träger oder Hilfsstoff enthält.

7. Pharmazeutische Zusammensetzung nach einem der Ansprüche 1 bis 6 zur Verwendung bei der Behandlung einer Krankheit, die mit einer anormalen Rekrutierung, Aktivierung oder Rekrutierung und Aktivierung von Leukozyten einhergeht, wobei die Krankheit ausgewählt wird aus der Gruppe bestehend aus Arthritis, Atherosklerose, Arteriosklerose, Restenose, Ischämie/Reperfusionsschaden, Diabetis Mellitus, Schuppenflechte, multiple Sklerose, chronisch-entzündliche Darmerkrankungen, Abstoßungsreaktionen gegen transplantierte Organe oder Gewebe, Graft-versus-Host-Erkrankung, Allergie und Asthma.

## Revendications

1. Composition pharmaceutique comprenant l'énantiomère (S) et l'énantiomère (R) du 4-(4-chloro-phényl)-1-{3-[7-(1-hydroxy-1-méthyléthyl)-11H-10-oxa-1-aza-dibenzo[a,d]cyclohepten-5-ylidène]-propyl}-3,3-diméthyl-pipéridin-4-ol, ou un sel physiologiquement acceptable de celui-ci, dans laquelle le rapport de l'énantiomère (S) à l'énantiomère (R) (p/p) dans la composition est d'au moins 2:1 environ.

2. Composition pharmaceutique selon la revendication 1, dans laquelle le rapport de l'énantiomère (S) à l'énantiomère (R) (p/p) dans la composition est d'au moins 5:1 environ.

3. Composition pharmaceutique selon la revendication 1, dans laquelle le rapport de l'énantiomère (S) à l'énantiomère (R) (p/p) dans la composition est d'au moins 10:1 environ.

4. Composition pharmaceutique selon la revendication 1, dans laquelle le rapport de l'énantiomère (S) à l'énantiomère (R) (p/p) dans la composition est d'au moins 20:1 environ.

5. Composition pharmaceutique selon la revendication 1, dans laquelle le rapport de l'énantiomère (S) à l'énantiomère (R) (p/p) dans la composition est d'au moins 50:1 environ.

6. Composition pharmaceutique selon l'une quelconque des revendications 1 à 5, comprenant un véhicule ou excipient physiologiquement acceptable.

7. Composition pharmaceutique selon l'une quelconque des revendications 1 à 6, destinée à être utilisée dans le traitement d'une maladie associée à un recrutement, une activation ou un recrutement et une activation aberrants des leucocytes, ladite maladie étant choisie dans le groupe comprenant l'arthrite, l'athérosclérose, l'artériosclérose, la resténose, une lésion d'ischémie/reperfusion, le diabète sucré, le psoriasis, la sclérose en plaques, les maladies inflammatoires chroniques de l'intestin, le rejet d'un organe ou tissu transplanté, la maladie du greffon contre l'hôte, l'allergie et l'asthme.
